(19) Europäisches Patentamt
European Patent Office
Office européen des brevets

(11) Numéro de publication: **0 202 164 B1**

# (12) FASCICULE DE BREVET EUROPEEN

(45) Date de publication du fascicule du brevet: **08.02.89**

(51) Int. Cl.⁴: **C 07 D 211/32**, A 61 K 31/445

(21) Numéro de dépôt: **86401000.4**

(22) Date de dépôt: **12.05.86**

(54) **Dérivés de (benzoyl-4 pipéridino)-2 phényl-1 alcanols, leur préparation et leur application en thérapeutique.**

(30) Priorité: **14.05.85 FR 8507270**

(43) Date de publication de la demande: **20.11.86 Bulletin 86/47**

(45) Mention de la délivrance du brevet: **08.02.89 Bulletin 89/6**

(84) Etats contractants désignés: **AT BE CH DE FR GB IT LI LU NL SE**

(56) Documents cités:
**EP-A- 0 109 317**
**FR-A- 2 227 868**

**Le dossier contient des informations techniques présentées postérieurement au dépôt de la demande et ne figurant pas dans le présent fascicule.**

(73) Titulaire: **SYNTHELABO, 58, rue de la Glacière, F-75621 Paris Cedex 13 (FR)**

(72) Inventeur: **Gaudilliere, Bernard, 28, rue Zilina, F-92000 Nanterre (FR)**
Inventeur: **Rousseau, Jean, 17Bis, Avenue de Montrouge, F-92340 Bourg-La-Reine (FR)**

(74) Mandataire: **Ludwig, Jacques et al, SYNTHELABO Service Brevets 58, rue de la Glacière, F-75621 Paris Cedex 13 (FR)**

EP 0 202 164 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet des dérivés de (benzoyl-4 pipéridino)-2 phényl-1 alcanols, leur préparation et leur application en thérapeutique.

Les composés de l'invention répondent à la formule I donnée dans le schéma 1 ci-après, formule dans laquelle

R représente un atome d'hydrogène ou un groupe méthyle,

$R_1$ représente

– soit un atome d'hydrogène ou un substituant unique en position 2, 3 ou 4, tel qu'un atome d'halogène, un groupe alkyle en $C_{1-4}$, alcoxy en $C_{1-4}$, hydroxy, benzyloxy, trifluorométhyle, cyano, nitro, amino, acétylamino, methylthio, methylsulfonyle ou aminosulfonyle,

– soit l'ensemble de 3 substituants qui sont le groupe méthoxy en 4 et deux groupes méthyles en 3 et 5, et

$R_2$ représente

– soit un atome d'hydrogène ou un substituant unique en position 3 ou 4 tel qu'un atome de fluor ou de chlore, un groupe méthyle ou un groupe méthoxy,

– soit l'ensemble de 3 substituants qui sont trois groupes méthoxy en 2, 4 et 6.

Les composés de l'invention peuvent se présenter sous forme de bases libres ou de sels d'addition à des acides.

Lorsque R désigne l'hydrogène, les molécules de formule I comportent un seul atome de carbone asymétrique. Ils peuvent donc se présenter sous la forme d'isomères optiques purs ou de leurs mélanges.

Lorsque R désigne un groupe méthyle, les molécules de formule I comportent deux atomes de carbone asymétriques vicinaux. Il y a donc deux formes diastéréoisomères, érythro et thréo, dont chacune comprend deux isomères optiques.

L'invention comprend chacune de ces formes pures, ainsi que leurs mélanges.

Des composés de structure proche de celle des composés de l'invention sont déjà connus, grâce à la demande de brevet européen n° 0 109 317.

Conformément à l'invention, on peut préparer les composés (I) par un procédé illustré par le schéma 1.

On fait d'abord réagir une cétone halogénée de formule II, dans laquelle R et $R_1$ sont tels que définis ci-dessus et X représente un atome de chlore ou de brome, avec une dioxolannylpipéridine de formule III, dans laquelle $R_2$ est tel que défini ci-dessus. La réaction s'effectue de préférence dans un solvant, et en présence d'une base. L'aminoacétone de formule IV ainsi obtenue est ensuite soumise à une réduction par exemple par un borohydrure de métal alcalin, pour donner un alcool de formule V.

Lorsque R représente un groupe méthyle, et selon les conditions de la réaction, il est possible de préparer sélectivement les isomères érythro ou thréo. Ainsi, en présence d'acide acétique, on obtient un mélange riche en isomère érythro, tandis qu'en l'absence d'acide on obtient un mélange riche en isomère thréo. Ces mélanges peuvent ensuite être purifiés, par exemple par chromatographie.

Finalement on soumet le composé (V) ainsi obtenu à l'action d'un acide comme l'acide chlorhydrique ou l'acide formique, ce qui donne directement le composé (I) sous forme de sel.

La cétone halogénée (II) de départ peut être obtenue par des procédés comme tels qu'ils sont décrits par exemple dans la demande de brevet européen n° 0 109 317.

La dioxolannylpipéridine de formule III peut être obtenue par acétalisation de la benzoyl-4 pipéridine correspondante, selon les méthodes analogues à celles décrites par exemple dans les demandes de brevet européens n° 0 013 612 et 0 070 053, ou dans le brevet des Etats Unis d'Amérique n° 4 335 127 ou encore dans la demande de brevet allemand n° 2 645 125.

Les composés de l'invention peuvent aussi être préparés selon un autre procédé illustré par le schéma 2.

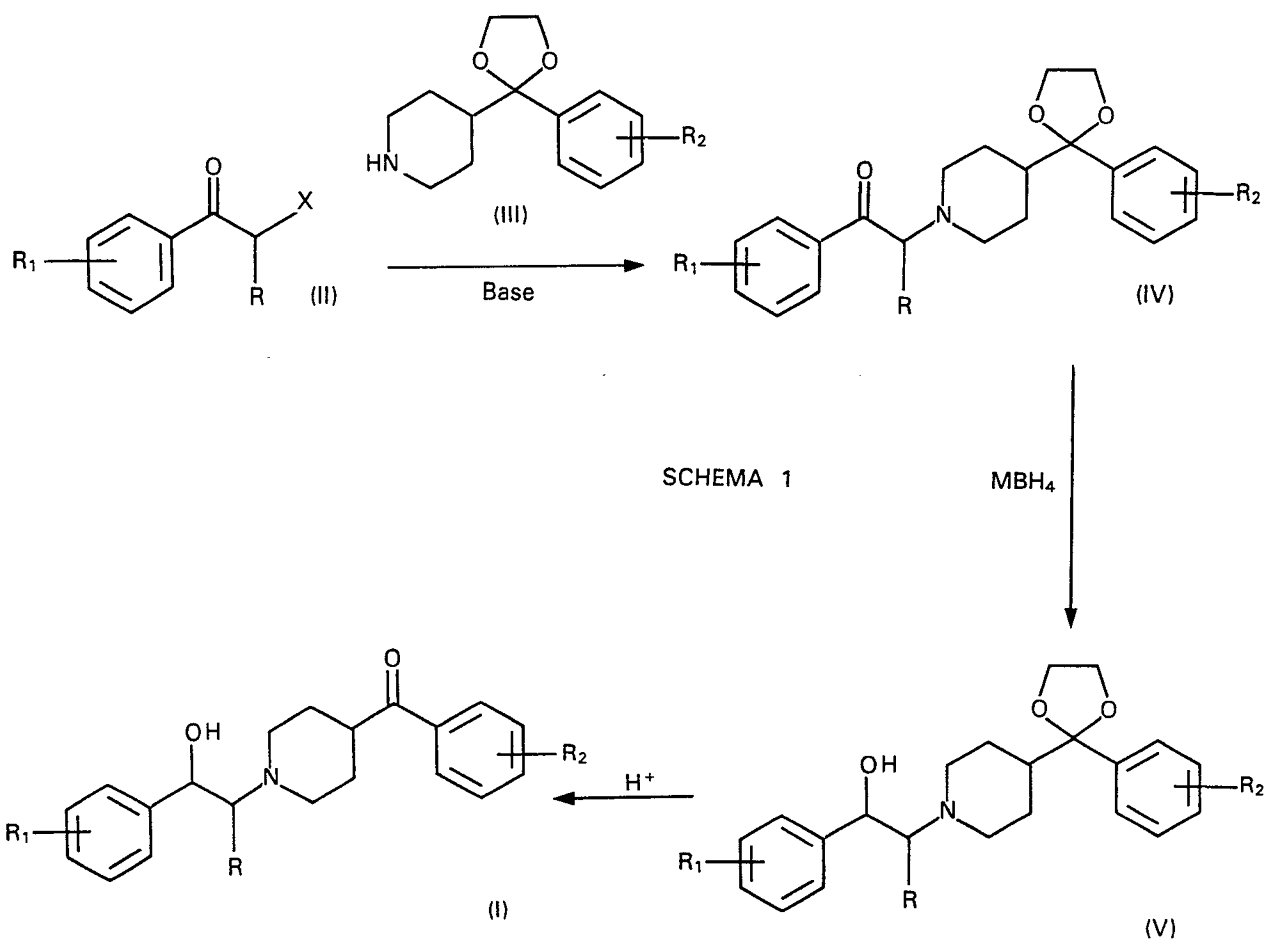


SCHEMA 1

SCHEMA 2

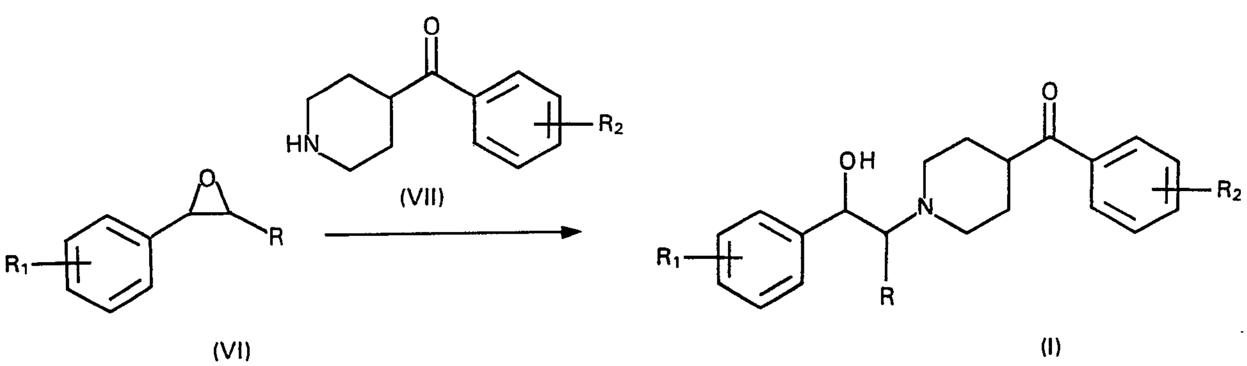


Le procédé consiste à additionner une benzoyl-4 pipéridine de formule VII sur un époxyde de formule VI. La réaction s'effectue par chauffage des réactifs à la température de reflux d'un solvant tel que l'éthanol.

L'époxyde (VI) peut être obtenu de diverses manières, soit, lorsque R = H, à partir du benzaldéhyde correspondant, par exemple par action d'iodure de triméthylsulfonium et de la potasse, ou à partir de l'α-bromo-acétophénone correspondante, par action d'un borohydrure alcalin puis de potasse, soit, lorsque R = H ou $CH_3$, à partir du styrène ou du propène-1 yl-1 benzène correspondant, par oxydation avec l'acide métachloroperbenzoïque.

Lorsque l'époxyde (VI) se trouve sous la forme d'un seul isomère optique, on peut effectuer une synthèse d'un composé final (I) optiquement actif.

Les benzoyl-4 pipéridines de formule VII peuvent être préparées par exemple par des méthodes telles que celles décrites par R.L. Duncan et coll., dans

J. Med. Chem., 1970, 13,1-6, ou par P. Manoury dans la demande de certificat d'addition français n° 2 408 602.

Enfin, les composés dans la formule (I) desquels $R_1$ représente un groupe hydroxy peuvent être obtenus par débenzylation des composés (I) où $R_1$ représente un groupe benzyloxy, et les composés dans la formule (I) desquels $R_1$ représente un groupe amino peuvent être obtenus par réduction des composés (I), ou de leurs précurseurs, dans la formule desquels $R_1$ représente un groupe nitro.

Les exemples qui vont suivre illustrent de manière détaillée la préparation de quelques composés selon l'invention.

Les microanalyses et les spectres IR et RMN de ces composés confirment leur structure chimique.

*Exemple 1*

[(Fluoro-4 benzoyl)-4 pipéridino]-2 phényl-1 éthanol.

Dans un erlenmeyer de 250 ml on introduit 2,4 g (0,02 mole) d'oxyde de styrène, 4,9 g (0,02 mole) de chlorhydrate de (fluoro-4 benzoyl)-4 pipéridine, 2,8 g de carbonate de potassium et 50 ml d'éthanol, et on chauffe le mélange au reflux pendant 3 h.

On chasse le solvant, on reprend le résidu avec de l'eau et on l'extrait à l'éther.

Les phases organiques réunies, lavées, séchées et évaporées livrent un produit cristallisé qu'on chromatographie sur colonne de silice en éluant avec un mélange 96/4 de chloroforme/méthanol.

On reprend la base ainsi purifiée avec 92 ml d'alcool isopropylique chlorhydrique 0,1 N, on évapore l'alcool et on recristallise le résidu dans de l'alcool isopropylique.

Point de fusion du chlorhydrate: 218°C.

*Exemple 2*

[(Fluoro-4 benzoyl)-4 pipéridino]-2 (chloro-4 phényl)-1 éthanol.

On dissout 3,85 g (0,025 mole) d'oxyde de chloro-4' styrène et 5,4 g (0,026 mole) de (fluoro-4 benzoyl)-4 pipéridine dans 100 ml d'éthanol anhydre, et on chauffe au reflux pendant 4 h.

On chasse l'alcool sous vide et on obtient un résidu huileux qui cristallise lorsqu'on le triture dans l'éther. On essore les cristaux, on évapore l'éther sous vide, et on obtient une deuxième fraction de produit par trituration du résidu solide dans du méthanol.

On en prépare et on recristallise le chlorhydrate comme indiqué à l'exemple 1.

Point de fusion du chlorhydrate: 221-223°C.

*Exemple 3*

[(Fluoro-4 benzoyl)-4 pipéridino]-2 (méthyl-3 phényl)-1 éthanol

Dans un ballon de 250 ml on introduit 1,34 g (0,01 mole) d'oxyde de méthyl-3' styrène, 2,43 g (0,01 mole) de chlorhydrate de (fluoro-4 benzoyl)-4 pipéridine, 1,40 g de carbonate de potassium et 50 ml d'éthanol.

On chauffe au reflux pendant 3 h, on évapore l'alcool, on reprend le résidu par de l'eau et on l'extrait par de l'acétate d'éthyle. On réunit les phases organiques, on les lave, on les sèche et on chasse le solvant. L'huile résiduelle cristallise.

On la purifie par chromatographie et on en prépare le chlorhydrate comme indiqué dans l'exemple 1, la recristallisation finale étant toutefois effectuée dans un mélange propanol-2/éthanol.

Point de fusion du chlorhydrate: 226°C.

*Exemple 4*

[(Fluoro-4 benzoyl)-4 pipéridino]-2 (méthoxy-4 phényl)-1 éthanol.

a) {[(fluoro-4 phényl)-2 dioxolanne-1,3-yl-2]-4 pipéridinyl-1}-2(méthoxy-4 phényl)-1 éthanone.

On chauffe au reflux pendant 4 h un mélange de 4,4 g (0,02 mole) de bromo-2 (méthoxy-4 phényl)-1 éthanone, 5,75 g (0,02 mole) de chlorhydrate de (fluoro-4 phényl)-2 (pipéridinyl-4)-2 dioxolanne-1,3, 2,76 g de carbonate de potassium et 100 ml d'acétonitrile.

On chasse le solvant, on reprend le résidu avec de l'eau et de l'ammoniaque diluée et on l'extrait par de l'acétate d'éthyle.

On réunit les phases organiques, on les lave, on les sèche sur sulfate de sodium et on évapore le solvant. On purifie le résidu par chromatographie en éluant avec un mélange 99/1 de chloroforme/méthanol.

b) {[(fluoro-4 phényl)-2 dioxolanne-1,3-yl-2]-4 pipéridinyl-1}-2 (méthoxy-4 phényl)-1 éthanol.

On agite un mélange de 3 g (0,0075 mole) du composé précédemment obtenu, 1,21 g (0,0225 mole) de borohydrure de potassium et 100 ml de méthanol pendant 2 h à température ambiante.

On évapore le solvant, on reprend le résidu avec un mélange d'eau et d'acétate d'éthyle, on sépare la phase organique, on la lave, on la sèche et on l'évapore. On recristallise le résidu dans l'éthanol.

Point de fusion: 120°C.

c) [(fluoro-4 benzoyl)-4 pipéridino]-2 (méthoxy-4 phényl)-1 éthanol.

On chauffe à 100°C, pendant 30 minutes au bain d'huile, un mélange de 1,5 g (0,0037 mole) du composé précédent et de 50 ml d'acide chlorhydrique 2N.

On laisse refroidir, on alcalinise le mélange par de l'ammoniaque diluée et on extrait la base par de l'acétate d'éthyle.

On lave, sèche et évapore le solvant, et on purifie le résidu par chromatographie en éluant avec un mélange 99/1 de chloroforme/méthanol.

Pour préparer le chlorhydrate on ajoute à la base une quantité stoechiométrique d'alcool isopropylique 0,1 N, soit 36,3 ml pour 1,3 g de base. Le sel cristallise par trituration. Après évaporation de l'alcool on le recristallise dans un mélange propanol-2/éthanol.

Point de fusion du chlorhydrate: 218°C.

*Exemple 5*

[(Fluoro-4 benzoyl)-4 pipéridino]-2 (fluoro-4 phényl)-1 propanol-1, forme érythro.

a) {[(fluoro-4 phényl)-2 dioxolanne-1,3 yl-2]-4 pipéridinyl-1}-2 (fluoro-4 phényl)-1 propanone-1.

On opère dans les conditions de l'exemple 4a) en partant de 9,24 g (0,04 mole) de bromo-2 (fluoro-4 phényl)-1 propanone-1, 10,05 g (0,04 mole) de (fluoro-4 phényl)-2 (pipéridinyl-4)-2 dioxolanne-1,3, et 5,56 g de carbonate de potassium dans 150 ml d'acétonitrile.

b) {[(fluoro-4 phényl)-2 dioxolanne-1,3 yl-2]-4 pipéridinyl-1}-2 (fluoro-4 phényl)-1 propanol-1.

On dissout 10 g du composé précédent dans un mélange de 150 ml de méthanol et 75 ml d'acide acétique, et on ajoute 6,7 g de borohydrure de potassium par petites portions. On agite le mélange pendant 2 h, et on le laisse reposer une nuit.

On évapore le solvant, on reprend l'huile résiduelle par de la glace et de l'ammoniaque diluée, et on l'extrait par du chlorure de méthylène. Après lavage, séchage et évaporation de la phase organique il reste un produit cristallin.

c) [(fluoro-4 benzoyl)-4 pipéridino]-2 (fluoro-4 phényl)-1 propanol-1, forme érythro.

On chauffe à 100°C, pendant 4 h au bain d'huile, une suspension de 9 g du composé précédent dans 300 ml d'acide chlorhydrique normal. Le chlorhydrate précipite. On l'essore, on le rince avec de l'eau puis de l'éther, on le sèche et on le recristallise dans l'éthanol.

Point de fusion du chlorhydrate: 250-252°C.

*Exemple 6*

[(Fluoro-4 benzoyl)- pipéridino]-2 (fluoro-4 phényl)-1 propanol-1, forme thréo.

a) {[(fluoro-4 phényl)-2 dioxolanne-1,3 yl-2]-4 pipéridinyl-1}-2 (fluoro-4 phényl)-1- propanol-1.

On dissout 10 g de la cétone obtenue selon l'exemple 5a dans 200 ml de méthanol, on ajoute 6,72 g de borohydrure de potassium et on agite le tout pendant 3 h à température ambiante.

On laisse reposer une nuit, on évapore le solvant, on alcalimise le résidu et on l'extrait par de l'acétate d'éthyle. Après lavage, séchage et évaporation de la phase organique on recueille un mélange où la forme thréo est majoritaire. On obtient la forme thréo pure par chromatographie sur silice en éluant avec un mélange 98/2 de chlorure de méthylène/méthanol.

b) [(fluoro-4 benzoyl)-4 pipéridino]-2 (fluoro-2 phényl)-1 propanol-1, forme thréo.

On procède comme à l'exemple 5c.

Point de fusion du chlorhydrate: 245-247°C.

*Exemple 7*

[(Fluoro-4 benzoyl)-4 pipéridino]-2 (hydroxy-4 phényl)-1 propanol-1, forme érythro.

a) (benzyloxy-4 phényl)-1 {[(fluoro-4 phényl)-2 dioxolanne-1,3 yl-2]-4 pipéridinyl-1}-2 propanol-1.

On chauffe au reflux pendant 4 h un mélange de 10,99 g (0,04 mole) de chloro-2 (benzyloxy-4 phényl)-1 propanone-1, 10,5 g (0,04 mole) de (fluoro-4 phényl)-2 (pipéridinyl-4)-2 dioxolanne-1,3, 5,52 g de carbonate de potassium et 150 ml d'acétonitrile.

En opérant comme indiqué à l'exemple 4a on isole une huile qu'on utilise telle quelle dans l'étape suivante.

b) (benzyloxy-4 phényl)-1 {[(fluoro-4 phényl)-2 dioxolanne-1,3 yl-2]-4 pipéridinyl-1}-2 propanol-1.

On dissout 16 g de l'huile précédemment obtenue dans un mélange de 150 ml de méthanol et 50 ml d'acide acétique. On refroidit la solution dans un bain de glace et on ajoute par petites portions 16 g de borohydrure de potassium.

On agite le mélange pendant 2 h, puis on évapore le solvant, on reprend le résidu cristallisé par de l'eau glacée, on ajoute de l'ammoniaque 3 N jusqu'à pH = 8, puis 250 ml d'acétate d'éthyle.

On agite 30 minutes, on essore le précipité formé, on le lave avec du pentane et on le sèche.

Point de fusion: 144-146°C.

c) (benzyloxy-4 phényl)-1 [(fluoro-4 benzoyl)-4 pipéridino]-2 propanol-1, forme érythro.

On met en suspension 2,5 g du composé précédent dans 100 ml d'acide chlorhydrique 1 N et on chauffe à 100°C pendant 6 h.

On filtre le chlorhydrate qui a précipité, on le rince à l'eau et on le recristallise dans un mélange éthanol/méthanol.

Point de fusion: 242-244°C.

d) [(fluoro-4 benzoyl)-4 pipéridino]-2 (hydroxy-4 phényl)-1 propanol-1, forme érythro.

On chauffe au reflux pendant 8 h un mélange de 5 g du composé précédent, 1 g de charbon palladié, 10 ml d'acide formique et 200 ml d'alcool isopropylique.

On sépare le catalyseur par filtration, on le rince avec du méthanol et on évapore le filtrat.

On reprend le résidu sec avec de l'eau glacée, on ajoute de l'ammoniaque 3 N jusqu'à pH = 8 et on extrait par du chloroforme.

Après décantation, lavage et séchage, on évapore la phase organique, ce qui donne un résidu blanc cristallisé.

A 4 g de la base ainsi obtenue on ajoute 103 ml de propanol-2 chlorhydrique 0,1 N, on agite, on évapore l'alcool et on recristallise le chlorhydrate dans l'éthanol.

Point de fusion: 212-214°C.

*Exemple 8*

R(–) [(Fluoro-4 benzoyl)-4 pipéridino]-2 phényl-1 éthanol.

Dans un ballon de 500 ml on chauffe au reflux, pendant 2 h, un mélange de 3,6 g (0,03 mole) de R(–)phényloxiranne, 7,3 g (0,03 mole) de chlorhydrate de (fluoro-4 benzoyl)-4 pipéridine, 8,3 g (0,06 mole) de carbonate de potassium et 200 ml d'éthanol.

Ensuite on évapore le solvant, on reprend le résidu avec de l'eau et on extrait la base organique au dichlorométhane. On lave la phase organique à l'eau, on la sèche sur sulfate de sodium et on l'évapore. On purifie le résidu par chromatographie sur silice en éluant avec un mélange 99,5/0,5 de chloroforme/méthanol, on prépare le chlorhydrate du solide purifié en ajoutant de l'alcool isopropylique chlorhydrique 0,1 N, et on le recristallise dans de l'alcool isopropylique.

Point de fusion du chlorhydrate: 205°C.

$[\alpha]_D^{20} = -34{,}15°$ (c = 0,5%, $CH_3OH$).

*Exemple 9*

S(+) [(Fluoro-4 benzoyl)-4 pipéridino]-2 phényl-1 éthanol

Dans un ballon de 500 ml on chauffe au reflux, pendant 3 h, un mélange de 3,24 g (0,027 mole) de S(+)phényloxiranne, 6,62 g (0, 027 mole) de chlorhydrate de (fluoro-4 benzoyl)-4 pipéridine, 7,5 g (0,055 mole) de carbonate de potassium et 250 ml d'éthanol.

Ensuite on évapore le solvant, on reprend le résidu avec un mélange d'eau et de dichlorométhane, on sépare la solution organique, on la lave à l'eau, on la sèche sur sulfate de sodium et on chasse le solvant. On purifie le résidu par chromatographie en éluant avec un mélange 99/1 de dichlorométhane/méthanol, on prépare le chlorhydrate du solide purifié en ajoutant de l'alcool isopropylique chlorhydrique 0,1 N, et on le recristallise dans de l'alcool isopropylique.

Point de fusion du chlorhydrate: 196-198°C.

$[\alpha]_D^{20} = +32,90°$ (c = 0,5%, $CH_3OH$).

*Exemple 10*

(Acétylamino-3 phényl)-1 ](fluoro-4 benzoyl)-4 pipéridino]-2 éthanol.

a) (Acétylamino-3 phényl)-1 éthanone.

A une suspension de 30 g 60,22 mole) d'(amino-3 phényl)-1 éthanone dans 200 ml de toluène, on ajoute à froid 50 ml d'anhydride acétique, on agite le mélange 1 h à température ambiante, puis on la chauffe 1 h à 60°C et on la laisse reposer la nuit. On essore le précipité obtenu, on le triture dans de l'éther et on le sèche.

b) (Acétylamino-3 phényl)-1 bromo-2 éthanone.

On chauffe au reflux un mélange de 17,7 g (0,1 mole) de la cétone précédente, 54,56 g (0,11 mole) de hydrotribromure de pyrrolidinone, 9,36 g (0,11 mole) de pyrrolidinone et 500 ml de tétrahydrofuranne.

On sépare un produit insoluble par filtration, en le rinçant avec du tétrahydrofuranne, on évapore le filtrat et on reprend l'huile résiduelle avec du dichlorométhane, on lave la solution à l'eau, on la sèche et on l'évapore. On triture l'huile résiduelle dans de l'éther. On obtient un produit cristallisé qu'on sèche à température ambiante et sous vide.

Point de fusion: 98°C.

c) (Acetylamino-3 phényl)-1 {[(fluoro-4 phényl)-2 dioxolanne-1,3 yl-2]-4 pipéridino}-2 éthanol.

On laisse réagir pendant 2 h à température ambiante un mélange de 8,9 g (0,035 mole) de la cétone bromée précédente, 8,7 g (0,035 mole) de [(fluoro-4 phényl)-2 dioxolanne-1,3 yl-2]-4 pipéridine et 7 g (0,08 mole) de carbonate de potassium dans 200 ml d'éthanol. On ajoute ensuite 12 g de borohydrure de potassium, puis 20 ml d'acide acétique pour favoriser la cinétique de la réaction, et on maintient l'agitation pendant 5 h.

On évapore le mélange, on le reprend avec de l'eau et de l'ammoniaque diluée et on extrait la base avec de l'acétate d'éthyle. On lave la solution organique à l'eau, on la sèche sur sulfate de sodium, on l'évapore et on purifie le résidu par chromatographie en éluant avec un mélange 98/2 de dichlorométhane/méthanol.

d) (Acétylamino-3 phényl)-1 [(fluoro-4 benzoyl)-4 pipéridino]-2 éthanol.

On dissout 2,4 g (0,0056 mole) du produit précédent dans 50 ml d'acide formique et on chauffe à 100°C pendant 3 h. Ensuite on jette le mélange sur de l'eau glacée, on ajoute de l'ammoniaque jusqu'à pH > 8, et on extrait la base organique par de l'acétate d'éthyle. On lave la phase organique à l'eau, on la sèche sur sulphate de magnésium, on chasse le solvant, et on purifie avec un mélange 98/2 de dichlorométhane/méthanol. Le produit purifié cristallise dans le pentane.

Point de fusion de la base libre: 158°C.

*Exemple 11*

(Amino-3 phényl)-1 [(fluoro-4 benzoyl)-4 pipéridino]-2 éthanol.

a) Bromo-2 (nitro-3 phényl)-1 éthanone.

On effectue une bromation de (nitro-3 phényl)-1 éthanone dans des conditions analogues à celles décrites dans l'exemple 10 b.

b) {[(fluoro-4 phényl)-2 dioxolanne-1,3 yl-2]-4 pipéridino}-2 (nitro-3 phényl)-1 éthanol.

On fait réagir le cétone bromée avec la [(fluoro-4 phényl)-2 dioxolanne-1,3 yl-2]-4 pipéridine dans des conditions analogues à celles décrites dans l'exemple 10 c.

c) {[(Fluoro-4 phényl)-2 dioxolanne-1,3 yl-2]-4 pipéridino}-2 (amino-3 phényl)-1 éthanol.

On effectue une réduction de 6 g (0,0144 mole) du composé précédent, dans 200 ml de méthanol, sous une pression de 0,35 MPa d'hydrogène en présence de 1 g de nickel de Raney. Après séparation du catalyseur par filtration, on évapore le filtrat, et on utilise le résidu solide tel que dans l'étape suivante.

d) (Amino-3 phényl)-1 [(fluoro-4 benzoyl)-4 pipéridino]-2 éthanol.

On introduit 6,3 g (0,0163 mole) du produit précédent dans 150 ml d'acide chlorhydrique 3N et on chauffe le mélange au bain d'huile à 100°C pendant 2 h. Il se forme une solution limpide qu'on jette sur 200 g de glace, on ajoute de l'ammoniaque jusqu'à pH > 8 et on extrait la base organique avec de l'acétate d'éthyle.

On lave la phase organique à l'eau, on la sèche, on l'évapore et on purifie le résidu par chromatographie en éluant avec un mélange 98/2 de chloroforme/méthanol.

On dissout 1,35 g de la base purifiée dans 25 ml d'éthanol et on ajoute 0,48 g d'acide benzoïque. Le benzoate précipite immédiatement; on le filtre et on le recristallise dans de l'alcool isopropylique.

Point de fusion du benzoate: 150°C.

*Exemple 12*

[(Chloro-4 benzoyl)-4 pipéridino]-2 (éthyl-4 phényl)-1 éthanol.

a) Bromo-2 (éthyl-4 phényl)-1 éthanone.

On effectue la bromation de l'(éthyl-4 phényl)-1 éthanone dans des conditions analogues à celles décrites dans l'exemple 10 b.

b) {[(Chloro-4 phényl)-2 dioxolanne-1,3 yl-2]-4 pipéridino}-2 (éthyl-4 phényl)-1 éthanol.

Dans 150 ml d'éthanol on introduit 5,34 g (0,02 mole) de [(chloro-4 phényl)-2 dioxolanne-1,3 yl-2]-4 pipéridine, 5,52 g (0,04 mole) de carbonate de potassium et 4,35 g (0,02 mole) de la cétone bromée précédente, et on chauffe au reflux pendant 1 h. On refroidit le milieu réactionnel par un bain de glace, on ajoute 10 ml d'acide acétique et, par petites quantités, 15 g de borohydrure de potassium.

Après retour du mélange à la température ambiante, on le laisse reposer une nuit.

On évapore le mélange, on reprend le résidu avec de l'eau et de l'ammoniaque et on l'extrait avec de dichlorométhane. On lave la phase organique à l'eau, on la sèche et on l'évapore. On purifie l'huile résiduelle par chromatographie en éluant avec un mélange 99/1 de dichlorométhane/méthanol.

c) [(Chloro-4 benzoyl)-4 pipéridino]-2 (éthyl-4 phényl)-1 éthanol.

On traite 5,8 g (0,014 mole) du composé précédent à 100°C par 200 ml d'acide chlorhydrique 3N, pendant 3 h, dans des conditions analogues à celles de l'exemple 11d.

On évapore la solution obtenue et on recristallise le chlorhydrate dans 75 ml d'alcool isopropylique.

Point de fusion du chlorhydrate: 252-254°C.

*Exemple 13*

[(Chloro-4 benzoyl)-4 pipéridino]-2 (fluoro-4 phényl)-1 propanol, forme thréo.

a) {[(Chloro-4 phényl)-2 dioxolanne-1,3 yl-2]-4 pipéridino}-2 (fluoro-4 phényl)-1 propanone.

Dans 280 ml d'acétonitrile on fait réagir 11,5 g (0,05 mole) de bromo-2 (fluoro-4 phényl)-1 propanone avec 13,38 g (0,05 mole) de (chloro-4 phényl)-2 dioxolanne-1,3 yl-2 -4 pipéridine, en présence de 13,9 g (0,1 mole) de carbonate de potassium, dans des conditions analogues à celles décrites dans les exemples 4a, 5a et 10c.

Après purification par chromatographie, on obtient une huile.

b) {[(Chloro-4 phényl)-2 dioxolanne-1,3 yl-2]-4 pipéridino}-2 (fluoro-4 phényl)-1 propanol, forme thréo.

On dissout 8 g (0,019 mole) de l'huile précédente dans 300 ml de méthanol, on ajoute 16 g de borohydrure de potassium, par petites quantités, on agite le mélange pendant 2 h et on le laisse reposer une nuit.

On chasse le méthanol, on reprend le résidu avec de l'eau et on l'extrait avec de l'acétate d'éthyle. Après lavage à l'eau, séchage et évaporation de la phase organique, on purifie le résidu par chromatographie en éluant avec un mélange 99/1 de dichlorométhane/methanol.

On obtient un solide cristallisé.

c) [(Chloro-4 benzoyl)-4 pipéridino]-2 (fluoro-4 phényl)-1 propanol, forme thréo.

On traite le composé précédent par de l'acide chlorhydrique 3N, dans des conditions analogues à celles décrites dans l'exemple 11d.

On évapore la solution obtenue et on recristallise le chlorhydrate dans l'alcool isopropylique.

Point de fusion du chlorhydrate: 243-245°C.

*Exemple 14*

[(Chloro-4 benzoyl)-4 pipéridino]-2 (fluoro-4 phényl)-1 propanol, forme érythro.

a) {[(Chloro-4 phényl)-2 dioxolanne-1,3 yl-2]-4 pipéridino}-2 (fluoro-4 phényl)-1 propanol, forme érythro.

On dissout 6 g de l'huile préparée selon l'exemple 13a dans un mélange de 300 ml de méthanol et 150 ml d'acide acétique et on ajoute 12 g de borohydrure de potassium par petites quantités. On agite encore pendant 2 h et on laisse reposer.

On évapore les solvants, on ajoute de l'eau et de l'ammoniaque jusqu'à pH basique, et on extrait avec de l'acétate d'éthyle. On lave, sèche et évapore la phase organique, et on purifie le résidu par chromatographie en éluant avec un mélange 99/1 de dichlorométhane/méthanol.

On obtient un solide cristallisé.

b) [(Chloro-4 benzoyl)-4 pipéridino]-2 (fluoro-4 phényl)-1 propanol, forme érythro.

On traite le composé précédent par de l'acide chlorhydrique 3N, dans des conditions analogues à celles décrites dans l'exemple 11 d.

On évapore la solution obtenue et on recristallise le chlorhydrate dans l'alcool isopropylique.

Point de fusion du chlorhydrate: 242°C.

Le tableau ci-après rassemble les structures et propriétés physiques de quelques composés selon l'invention.

TABLEAU

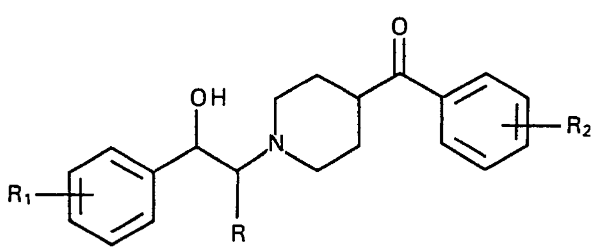

(I)

| Composé | R | $R_1$ | $R_2$ | Isomère | Sel(1) | F(°C) |
|---|---|---|---|---|---|---|
| 1 (Ex. 1) | H | H | 4-F | (±) | 10 | 218 |
| 2 (Ex. 9) | H | H | 4-F | (+) | 10 | 196-198 |

TABLEAU (suite)

| Composé | R | $R_1$ | $R_2$ | Isomère | Sel(1) | F(°C) |
|---|---|---|---|---|---|---|
| 3 (Ex. 8) | H | H | 4-F | (−) | 10 | 205 |
| 4 | H | 4-Cl | H | (±) | 10 | 211-213 |
| 5 (Ex. 2) | H | 4-Cl | 4-F | (±) | 10 | 221-223 |
| 6 | H | 4-Cl | $4\text{-}OCH_3$ | (±) | 10 | 210-212 |
| 7 | H | 4-Cl | $2,4,6\text{-}(OCH_3)_3$ | (±) | 10 | 201-202 |
| 8 | H | 3-Cl | 4-F | (±) | 10 | 179-181 |
| 9 | H | 2-Cl | 4-F | (±) | 10 | 212-214 |
| 10 | H | 4-F | H | (±) | 10 | 188 |
| 11 | H | 4-F | 4-F | (±) | 10 | 197-198 |
| 12 | H | 4-F | 3-F | (±) | 10 | 170-172 |
| 13 | H | 3-F | 4-F | (±) | 10 | 178-180 |
| 14 | H | 4-F | 4-Cl | (±) | 10 | 218-220 |
| 15 | H | $4\text{-}CF_3$ | 4-F | (±) | 10 | 226-228 |
| 16 | H | $3\text{-}CF_3$ | 4-F | (±) | 10 | 206-208 |
| 17 | H | $2\text{-}CH_3$ | 4-F | (±) | 10 | 206 |
| 18 (Ex. 3) | H | $3\text{-}CH_3$ | 4-F | (±) | 10 | 226 |
| 19 | H | $4\text{-}CH_3$ | 4-F | (±) | 10 | 240-242 |
| 20 | H | $4\text{-}CH_3$ | 3-F | (±) | 10 | 212 |
| 21 | H | $4\text{-}CH_3$ | H | (±) | 10 | 225-226 |
| 22 | H | $4\text{-}CH_3$ | $4\text{-}CH_3$ | (±) | 10 | 259-260 |
| 23 | H | $4\text{-}CH_3$ | $4\text{-}OCH_3$ | (±) | 10 | 228-229 |
| 24 | H | $3\text{-}C_2H_5$ | 4-F | (±) | 10 | 195 |
| 25 | H | $4\text{-}C_2H_5$ | 4-F | (±) | 10 | 242-244 |
| 26 (Ex. 12) | H | $4\text{-}C_2H_5$ | 3-Cl | (±) | 10 | 252-254 |
| 27 | H | $3\text{-}C_2H_5$ | 3-F | (±) | 10 | 179 |
| 28 | H | $4\text{-}nC_4H_9$ | 4-F | (±) | 10 | 252-254 |
| 29 (Ex.4) | H | $4\text{-}OCH_3$ | 4-F | (±) | 10 | 218 |
| 30 | H | $3\text{-}OCH_3$ | 4-F | (±) | 10 | 204 |
| 31 | H | 4-OH | 4-F | (±) | 10 | 185 |
| 32 | H | $4\text{-}OnC_4H_9$ | 4-F | (±) | 10 | 243-245 |
| 33 | H | $4\text{-}SCH_3$ | 4-F | (±) | 10 | 221-222 |
| 34 | H | $4\text{-}SO_2CH_3$ | 4-F | (±) | 00 | 186-187 |
| 35 | H | $4\text{-}NO_2$ | 4-F | (±) | 10 | 224-226 |
| 36 | H | $3\text{-}NO_2$ | 4-F | (±) | 10 | 214-215 |
| 37 | H | $4\text{-}NHCOCH_3$ | 4-F | (±) | 10 | 210 |
| 38 (Ex. 10) | H | $3\text{-}NHCOCH_3$ | 4-F | (±) | 00 | 158 |
| 39 (Ex. 11) | H | $3\text{-}NH_2$ | 4-F | (±) | 03 | 150 |
| 40 | H | $3\text{-}SO_2NH_2$ | 4-F | (±) | 10 | 238 |
| 41 | H | 4-CN | 4-F | (±) | 10 | 235-237 |
| 42 | $CH_3$ | H | 4-F | (±) érythro | 10 | 256-258 |
| 43 | $CH_3$ | H | 4-F | (±) thréo | 10 | 216-218 |
| 44 | $CH_3$ | 4-Cl | 4-F | (±) érythro | 10 | 235-236 |
| 45 | $CH_3$ | 4-Cl | 4-F | (±) thréo | 10 | 236-237 |
| 46 | $CH_3$ | 3-Cl | 4-F | (±) érythro | 10 | 228-229 |
| 47 | $CH_3$ | 3-Cl | 4-F | (±) thréo | 10 | 248-249 |
| 48 | $CH_3$ | 2-Cl | H | (±) érythro | 14 | 192 |
| 49 | $CH_3$ | 2-Cl | 4-F | (±) érythro | 14 | 198 |
| 50 (Ex. 5) | $CH_3$ | 4-F | 4-F | (±) érythro | 10 | 250-252 |
| 51 (Ex. 6) | $CH_3$ | 4-F | 4-F | (±) thréo | 10 | 245-247 |

TABLEAU (suite)

| Composé | R | $R_1$ | $R_2$ | Isomère | Sel(1) | F(°C) |
|---|---|---|---|---|---|---|
| 52 (Ex. 14) | $CH_3$ | 4-F | 4-Cl | (±) érythro | 10 | 242 |
| 53 (Ex. 13) | $CH_3$ | 4-F | 4-Cl | (±) thréo | 10 | 243-245 |
| 54 | $CH_3$ | 4-F | 4-$CH_3$ | (±) érythro | 10 | 256-257 |
| 55 | $CH_3$ | 4-$CF_3$ | 4-F | (±) érythro | 10 | 264-265 |
| 56 | $CH_3$ | 4-$CF_3$ | 4-F | (±) thréo | 10 | 232-234 |
| 57 | $CH_3$ | 4-$CH_3$ | 4-F | (±) érxthro | 10 | 265 |
| 58 | $CH_3$ | 4-$CH_3$ | 4-F | (±) thréo | 10 | 245 |
| 59 | $CH_3$ | 3-$CH_3$ | 4-F | (±) érythro | 10 | 242-244 |
| 60 | $CH_3$ | 3-$CH_3$ | 4-F | (±) thréo | 10 | 232-234 |
| 61 | $CH_3$ | 4-$C_2H_5$ | 4-F | (±) érythro | 10 | 266-268 |
| 62 | $CH_3$ | 4-$C_2H_5$ | 4-F | (±) thréo | 10 | 238-240 |
| 63 (Ex. 7d) | $CH_3$ | 4-OH | 4-F | (±) érythro | 10 | 212-214 |
| 64 (Ex. 7c) | $CH_3$ | 4-$OCH_2C_6H_5$ | 4-F | (±) érythro | 10 | 242-244 |
| 65 | $CH_3$ | 4-$NHCOCH_3$ | 4-F | (±) thréo | 10 | 223-225 |
| 66 | $CH_3$ | 4-CN | 4-F | (±) érythro | 10 | 256-258 |
| 67 | $CH_3$ | 4-CN | 4-F | (±) thréo | 10 | 246-247 |
| 68 | $CH_3$ | 4-$OCH_3$ 3,5-$(CH_3)_2$ | 4-F | (±) érythro | 10 | 262-263 |
| 69 | $CH_3$ | 4-$OCH_3$ 3,5-$(CH_3)_2$ | 4-$CH_3$ | (±) érythro | 10 | 261-262 |

(1) 00: base libre 03: benzoate 10: chlorhydrate 14: maléate

Les composés de l'invention ont été soumis à des essais pharmacologiques.

Leur toxicité a été déterminée chez des souris de souche CDI par une méthode graphique. Les doses létales 50 ($DI_{50}$) sont pour la plupart supérieures à 100 mg/kg par voie intrapéritonéale.

Les composés de l'invention ont été soumis au test de l'ischémie cérébrale complète. L'ischémie est due à un arrêt cardiaque induit par une injection intraveineuse rapide de chlorure de magnésium.

Dans ce test on mesure le «temps de survie», c'est-à-dire l'intervalle entre le moment de l'injection de chlorure de magnésium et le dernier mouvement respiratoire observable de chaque souris.

Ce dernier mouvement est considéré comme l'indice ultime d'une fonction du système nerveux central.

L'arrêt respiratoire apparaît approximativement 19 secondes après l'injection de chlorure de magnésium.

Des souris mâles (Charles River CDI) sont étudiées par groupes de 10.

Les souris sont nourries et abreuvées ad libitum avant les essais. Le temps de survie est mesuré 10 minutes après l'administration intrapéritonéale des composés de l'invention.

Les résultats sont donnés sous la forme de la différence entre le temps de survie mesuré dans un groupe de 10 souris ayant reçu le composé et le temps de survie mesuré dans un groupe de 10 souris ayant reçu le liquide véhicule.

Les rapports entre les modifications dans le terme de survie et la dose du composé sont enregistrés graphiquement selon une courbe semilogarithmique.

Cette courbe permet le calcul de la dose effective 3 secondes ($DE_{3''}$), c'est-à-dire la dose (en mg/kg) qui produit une augmentation de 3 secondes du temps de survie par rapport au groupe témoin de 10 souris non traitées.

Une augmentation de 3 secondes dans le temps de survie est à la fois significative statistiquement et reproductible.

Les $DE_{3''}$ des composés de l'invention vont de 0,5 à 14 mg/kg par voie i.p.

Les composés de l'invention également ont fait l'objet d'un essai de déplacement de la liaison (binding) du spiropéridol sur les récepteurs sérotoninergiques (5-$HT_2$) du cortex cérébral du rat.

Pour cet essai on prélève les cerveaux de rats, on en dissèque le cortex et on l'homogénéise à 0°C dans 50 volumes d'un mélange contenant, par litre, 50 millimoles de tampon Tris/HCl à pH = 7,4, 120 millimoles de chlorure de sodium et 5 millimoles de chlorure de potassium. On centrifuge le mélange homogène à 40000 × g pendant 10 minutes puis, à deux reprises, on récupère le culot, on le lave en le mettant en suspension dans le même mélange tampon, on l'homogénéise à nouveau et on le centrifuge. Pour terminer on dilue le culot final dans le même mélange tampon à raison de 10 mg de tissu humide pour 1 ml de tampon.

On soumet alors le tissu à une incubation préalable de 5 minutes à 37°C en présence de 0,1% d'acide ascorbique et 10 micromoles/l de pargyline, puis à une incubation de 20 minutes à 37°C en présence de $^3$H-spiropéridol (activité spécifique: 25,6 Ci/millimole) à la concentration de 0,3 nanomole/l et de composé à étudier à des concentrations allant de 0,0001 à 100 micromoles/l.

On prélève des aliquots de 1 ml que l'on filtre sous vide, on lave les filtres deux fois avec 5 ml de tampon froid et on les sèche. La radioactivité est mesurée dans le toluène en présence de 5 g/l de diphényl-2,5 oxazole (PPO) et 0,1 g/l de bis-(phényl-5 oxazolyl-2)--1,4 benzène (POPOP).

Pour évaluer l'activité des composés on établit la courbe du pourcentage d'inhibition de la liaison spécifique de $^3$H-spiropéridol en fonction de la concentration en drogue déplaçante. On détermine graphiquement la concentration $IC_{50}$, concentration qui inhibe 50% de la liaison spécifique. La liasion spécifique est définie comme étant la liaison déplacée par 100 micromoles/l de 5-HT.

Les concentrations $IC_{50}$ des composés de l'invention se situent entre 0,01 et 3,6 micromoles/l.

Enfin les composés de l'invention ont été soumis à un essai de déplacement de la liaison de la prazosine (agent antihypertenseur) sur les récepteurs $alpha_1$ du cortex cérébral du rat.

On homogénéise des cortex de rats dans 30 volumes d'un tampon Tris/HCl (50 millimoles/l, pH = 7,5) pendant 30 secondes, puis on effectue une centrifugation à 45000 × g pendant 10 minutes. On remet le culot en suspension dans le tampon, et on l'homogénéise et le centrifuge une deuxième fois dans les mêmes conditions.

Finalement on met les membranes en suspension dans 100 volumes du même tampon pour obtenir une concentration finale de 10 mg de tissu humide par ml (soit 0,55 mg de protéine par ml).

On fait ensuite incuber, pendant 30 minutes à 25°C, des quantités de 1 ml de suspension en présence de 0,5 nanomole/l de $^3$H-prazosine (activité spécifique: 25,4 Ci/millimole) et de composés à étudier à des concentrations de 0,0001 à 100 micromoles/l.

Après incubation on dilue 0,45 ml de chaque mélange dans 3 ml de tampon, on filtre rapidement et on lave deux fois le résidu avec 5 ml de tampon froid. On sèche les filtres et on mesure la radioactivité par spectrométrie de scintillation. On établit pour chaque composé la courbe du pourcentage d'inhibition de la liaison de $^3$H-prazosine en fonction de la concentration en drogue déplaçante, et on détermine graphiquement la concentration $IC_{50}$, concentration qui inhibe 50% de la liaison spécifique.

La liaison spécifique est définie comme étant la liaison déplacée en présence de 10 micromoles/l de phentolamine.

Les concentrations $IC_{50}$ des composés de l'invention vont de 0,001 à plus de 10 micromoles/l.

L'étude pharmocologique des composés de l'invention montre qu'ils ont une activité anti-ischémique et qu'ils peuvent être utilisés en thérapeutique pour le traitement des troubles de la vigilance, en particulier pour lutter contre les troubles du comportement imputables à des dommages vasculaires cérébraux et à la sclérose cérébrale en gériatrie, ainsi que pour le traitement des encéphalopathies métaboliques et de la migraine, le traitement des troubles vasculaires cardiaques et périphériques, et pour le traitement des états dépressifs.

L'invention comprend par conséquent, toutes compositions pharmaceutiques renfermant les composés et/ou leurs sels comme principes actifs, en association avec tous excipients appropriés à leurs administration, en particulier par voie orale ou parentérale.

Les voies d'administration peuvent être les voies orale et parentérale.

La posologie quotidienne peut aller de 1 à 100 mg par voie parentérale et de 5 à 500 mg par voie orale.

**Revendications pour les Etats Contractants:**
BE, CH, DE, FR, GB, IT, LI, LU, NL, SE

1. Composé, sous forme d'isomères optiques purs ou de leurs mélanges, caractérisés en ce qu'ils répondent à la formule générale I

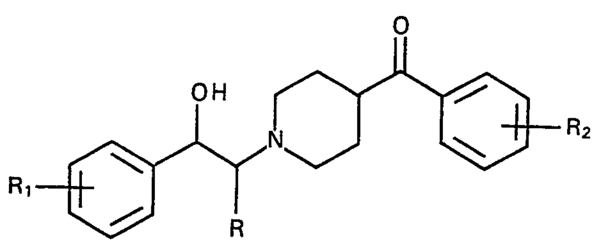

(I)

dans laquelle

R représente un atome d'hydrogène ou un groupe méthyle,

$R_1$ représente

– soit un atome d'hydrogène ou un substituant unique en position 2, 3 ou 4, tel qu'un atome d'halogène, un groupe alkyle en $C_{1-4}$, alcoxy en $C_{1-4}$, hydroxy, benzyloxy, trifluorométhyle, cyano, nitro, amino, acétylamino, methylthio, methylsulfonyle ou aminosulfonyle,

– soit l'ensemble de 3 substituants qui sont le groupe méthoxy en 4 et deux groupes méthyles en 3 et 5, et

$R_2$ représente

– soit un atome d'hydrogène ou un substituant unique en position 3 ou 4 tel qu'un atome de fluor ou de chlore, un groupe méthyle ou un groupe méthoxy,

– soit l'ensemble de 3 substituants qui sont trois groupes méthoxy en 2, 4 et 6,

ainsi que leurs sels d'addition à des acides acceptables en pharmacologie.

2. Composé selon la revendication 1, caractérisé en ce que $R_1$ représente un atome d'halogène ou un groupe alkyle, et $R_2$ représente un atome de fluor.

3. Procédé de préparation des composés selon la revendication 1, caractérisé en ce qu'on fait réagir une cétone halogénée de formule II

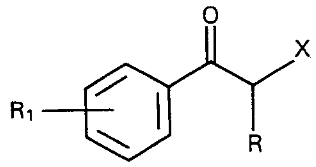

(II)

dans laquelle R et $R_1$ sont tels que définis dans la revendication 1 et X représente un atome de chlore ou de brome, avec une dioxolannylpipéridine de formule III

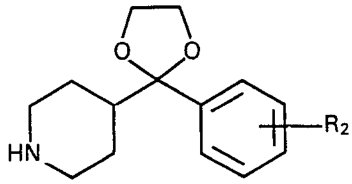

(III)

dans laquelle $R_2$ est tel que défini dans la revendication 1, puis on soumet le composé obtenu, de formule IV,

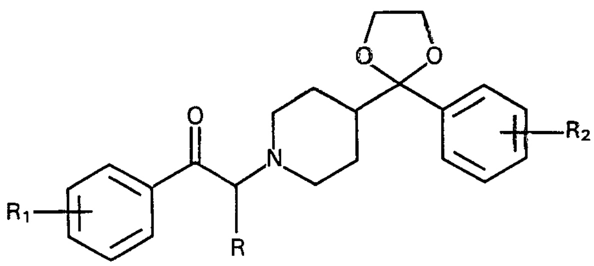

(IV)

à une réduction pour obtenir un composé de formule V

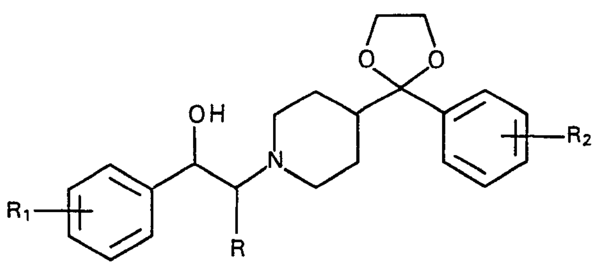

(V)

que l'on soumet finalement à l'action d'un acide.

4. Procédé selon la revendication 3, caractérisé en ce qu'on fait réagir la cétone halogénée (III) avec la dioxolannylpipéridine (IV) sous forme de sel, dans un solvant et en présence d'une base.

5. Procédé selon la revendication 3, caractérisé en ce qu'on réduit le composé (IV) au moyen d'un borohydrure de métal alcalin.

6. Procédé selon la revendication 5, caractérisé en ce qu'on opère en présence d'acide acétique.

7. Procédé selon la revendication 5, caractérisé en ce qu'on opère en l'absence d'acide.

8. Procédé de préparation des composés selon la revendication 1, caractérisé en ce qu'on additionne une benzoyl-4 pipéridine de formule VII

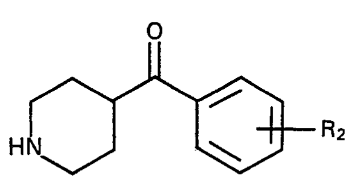

(VII)

dans laquelle $R_2$ est tel que défini dans la revendication 1, sur un époxyde de formule VI

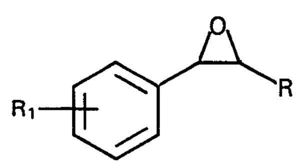

(VI)

dans laquelle R et $R_1$ sont tels que définis dans la revendication 1.

9. Médicament, caractérisé en ce qu'il est constitué d'un composé selon l'une des revendications 1 et 2.

10. Composition pharmaceutique, caractérisée en ce qu'elle contient un composé selon l'une des revendications 1 et 2, en association avec un excipient approprié.

**Revendications pour l'Etat Contractant:** AT

Procédé de préparation de composés répondant à la formule générale I

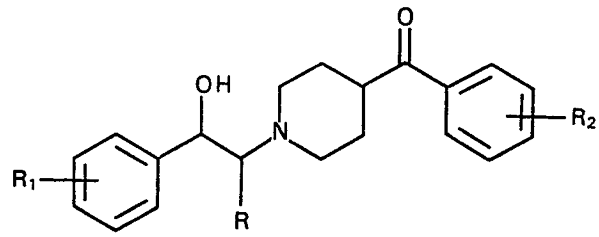

(I)

dans laquelle

R représente un atome d'hydrogène ou un groupe méthyle,

$R_1$ représente
– soit un atome d'hydrogène ou un substituant unique en position 2, 3 ou 4, tel qu'un atome d'halogène, un groupe alkyle en $C_{1-4}$, alcoxy en $C_{1-4}$, hydroxy, benzyloxy, trifluorométhyle, cyano, nitro, amino, acétylamino, methylthio, methylsulfonyle ou aminosulfonyle,
– soit l'ensemble de 3 substituants qui sont le groupe méthoxy en 4 et deux groupes méthyles en 3 et 5, et

$R_2$ représente

– soit un atome d'hydrogène ou un substituant unique en position 3 ou 4 tel qu'un atome de fluor ou de chlore, un groupe méthyle ou un groupe méthoxy,
– soit l'ensemble de 3 substituants qui sont trois groupes méthoxy en 2, 4 et 6,
procédé caractérisé en ce qu'on fait réagir une cétone halogénée de formule II

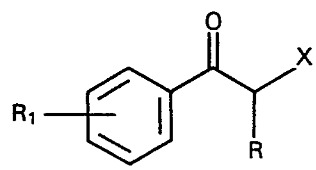

(II)

dans laquelle R et $R_1$ sont tels que définis ci-dessus et X représente un atome de chlore ou de brome, avec une dioxolannylpipéridine de formule III

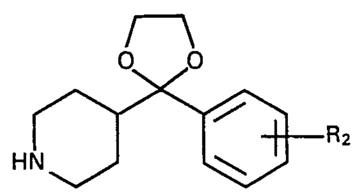

(III)

dans laquelle $R_2$ est tel que défini ci-dessus, puis on soumet le composé obtenu, de formule IV,

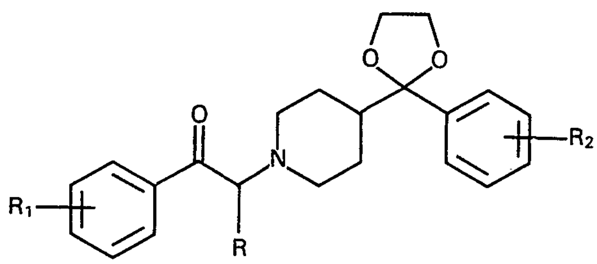

(IV)

à une réduction pour obtenir un composé de formule V

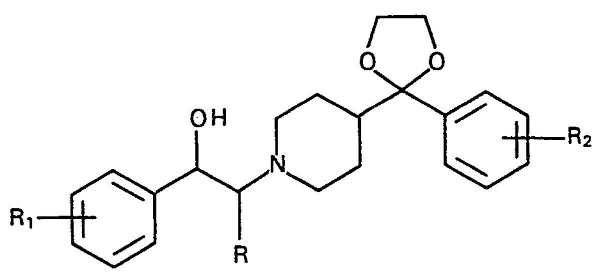

(V)

que l'on soumet finalement à l'action d'un acide.

2. Procédé selon la revendication 1, caractérisé en ce qu'on fait réagir la cétone halogénée (III) avec la dioxolannylpipéridine (IV) sous forme de sel, dans un solvant et en présence d'une base.

3. Procédé selon la revendication 2, caractérisé en ce qu'on réduit le composé (IV) au moyen d'un borohydrure de métal alcalin.

4. Procédé selon la revendication 3, caractérisé en ce qu'on opère en présence d'acide acétique.

5. Procédé selon la revendication 3, caractérisé en ce qu'on opère en l'absence d'acide.

6. Procédé de préparation de composé répondant à la formule générale I

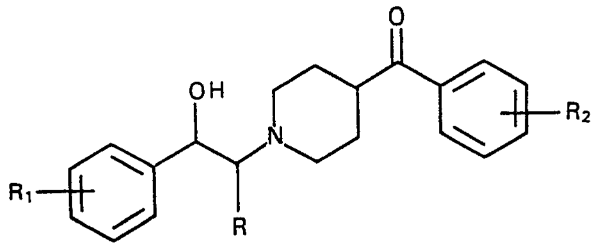

(I)

dans laquelle

R représente un atome d'hydrogène ou un groupe méthyle,

$R_1$ représente

– soit un atome d'hydrogène ou un substituant unique en position 2, 3 ou 4, tel qu'un atome d'halogène, un groupe alkyle en $C_{1-4}$, alcoxy en $C_{1-4}$, hydroxy, benzyloxy, trifluorométhyle, cyano, nitro, amino, acétylamino, methylthio, methylsulfonyle ou aminosulfonyle,

– soit l'ensemble de 3 substituants qui sont le groupe méthoxy en 4 et deux groupes méthyles en 3 et 5, et

$R_2$ représente

– soit un atome d'hydrogène ou un substituant unique en position 3 ou 4 tel qu'un atome de fluor ou de chlore, un groupe méthyle ou un groupe méthoxy,

– soit l'ensemble de 3 substituants qui sont trois groupes méthoxy en 2, 4 et 6,

procédé caractérisé en ce qu'on additionne une benzoyl-4 pipéridine de formule VII

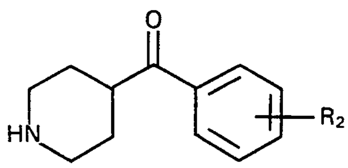

(VII)

dans laquelle $R_2$ est tel que défini ci-dessus, sur un époxyde de formule VI

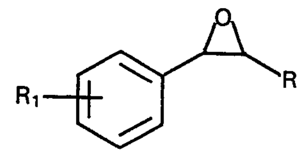

(VI)

dans laquelle R et $R_1$ sont tels que définis ci-dessus.

**Patentansprüche für die Vertragstaaten:**
BE, CH, DE, FR, GB, IT, LI, LU, NL, SE

1. Verbindungen in Form der reinen optischen Isomeren oder deren Mischungen, dadurch gekennzeichnet, dass sie der allgemeinen Formel I entsprechen,

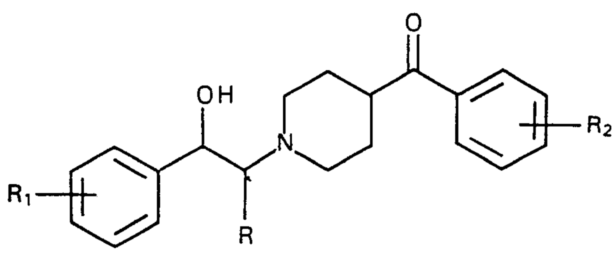

(I)

in welcher

R für ein Wasserstoffatom oder eine Methylgruppe,

$R_1$

– entweder für ein Wasserstoffatom oder einen einzigen Substituenten in der Stellung 2, 3 oder 4, wie ein Halogenatom, eine $C_{1-4}$-Alkyl-, $C_{1-4}$-Alkoxy-, Hydroxy-, Benzyloxy-, Trifluormethyl-, Cyano-, Nitro-, Amino-, Acetylamino-, Methylthio-, Methylsulfonyl- oder Aminosulfonylgruppe

– oder für insgesamt 3 Substituenten steht, die die Methoxygruppe in 4-Stellung und zwei Methylgruppen in den Stellungen 3 und 5 bedeuten,

und $R_2$

– .entweder ein Wasserstoffatom oder einen einzigen Substituenten in der Stellung 3 oder 4, wie ein Fluor- oder Chloratom, eine Methylgruppe oder eine Methoxygruppe

– oder insgesamt drei Substituenten darstellt, die drei Methoxygruppen in den Stellungen 2, 4 und 6 bedeuten,

sowie deren Additionssalze mit pharmakologisch verwendbaren Säuren.

2. Verfahren nach Anspruch 1, dadurch gekennzeichnet, dass $R_1$ für ein Wasserstoffatom oder eine Alkylgruppe und $R_2$ für ein Fluoratom steht.

3. Verfahren zur Herstellung von Verbindungen nach Anspruch 1, dadurch gekennzeichnet, dass man ein halogeniertes Keton der Formel II

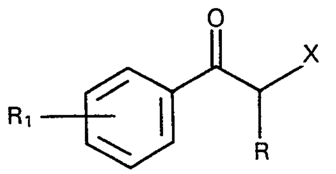

(II),

in welcher R und $R_1$ die in Anspruch 1 genannte Bedeutung haben und X für ein Chlor- oder Bromatom steht, mit einem Dioxolannylpiperidin der Formel III

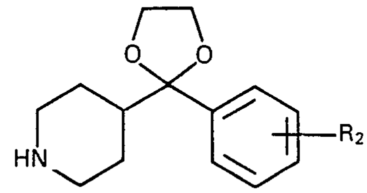

(III),

in welcher $R_2$ die in Anspruch 1 genannte Bedeutung hat, umsetzt, und die erhaltene Verbindung der Formel IV

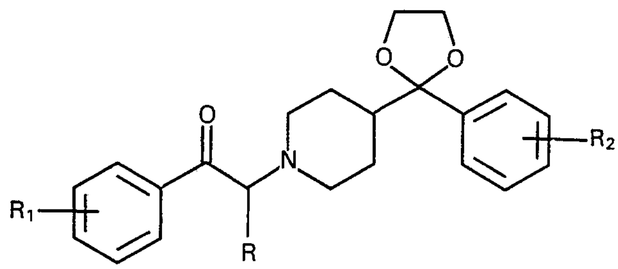

(IV)

einer Reduktion unterwirft, um eine Verbindung der Formel V

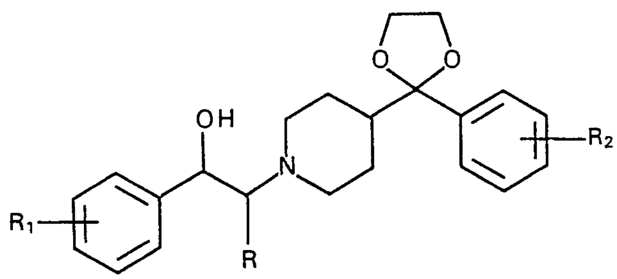

(V)

zu erhalten, die man schliesslich der Einwirkung einer Säure aussetzt.

4. Verfahren nach Anspruch 3, dadurch gekennzeichnet, dass man das halogenierte Keton (III) mit dem Dioxolannylpiperidin (IV) in Form des Salzes in einem Lösungsmittel und in Gegenwart einer Base umsetzt.

5. Verfahren nach Anspruch 3, dadurch gekennzeichnet, dass man die Verbindung (IV) mit Hilfe eines Alkalimetallborhydrids reduziert.

6. Verfahren nach Anspruch 5, dadurch gekennzeichnet, dass man in Gegenwart von Essigsäure arbeitet.

7. Verfahren nach Anspruch 5, dadurch gekennzeichnet, dass man in Abwesenheit einer Säure arbeitet.

8. Verfahren zur Herstellung von Verbindungen nach Anspruch 1, dadurch gekennzeichnet, dass man ein 4-Benzoylpiperidin der Formel VII

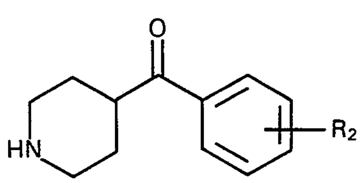

(VII),

in welcher $R_2$ die in Anspruch 1 genannte Bedeutung hat, zu einem Epoxid der Formel VI

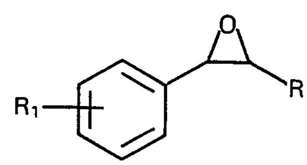

(VI),

worin R und $R_1$ die in Anspruch 1 angegebene Bedeutung haben, zusetzt.

9. Arzneimittel, dadurch gekennzeichnet, dass es aus einer Verbindung nach einem der Ansprüche 1 oder 2 besteht.

10. Pharmazeutische Zusammensetzung, dadurch gekennzeichnet, dass sie eine Verbindung nach einem der Ansprüche 1 oder 2 in Kombination mit einem geeigneten Bindemittel enthält.

**Patentansprüche für den Vertragsstaat:** AT

1. Verfahren zur Herstellung von Verbindungen, die der allgemeinen Formel I entsprechen

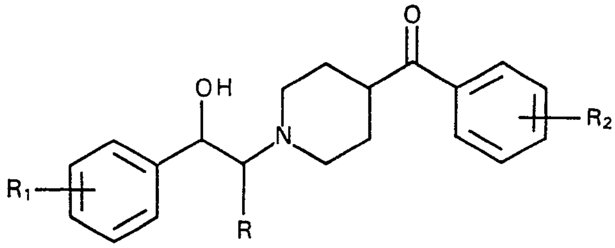

(I),

in welcher

R für ein Wasserstoffatom oder eine Methylgruppe,

$R_1$

– entweder für ein Wasserstoffatom oder einen einzigen Substituenten in der Stellung 2, 3 oder 4, wie ein Halogenatom, eine $C_{1-4}$-Alkyl-, $C_{1-4}$-Alkoxy-, Hydroxy-, Benzyloxy-, Trifluormethyl-, Cyano-, Nitro-, Amino-, Acetylamino-, Methylthio-, Methylsulfonyl- oder Aminosulfonylgruppe

– oder für insgesamt 3 Substituenten steht, die die Methoxygruppe in 4-Stellung und zwei Methylgruppen in den Stellungen 3 und 5 bedeuten,

und $R_2$

– entweder ein Wasserstoffatom oder einen einzigen Substituenten in der Stellung 3 oder 4, wie ein Fluor- oder Chloratom, eine Methylgruppe oder eine Methoxygruppe

– oder insgesamt 3 Substituenten darstellt, die 3 Methoxygruppen in den Stellungen 2, 4 und 6 bedeuten,

welches Verfahren dadurch gekennzeichnet ist, dass man ein halogeniertes Keton der Formel II

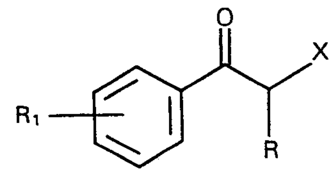

(II),

in welcher R und $R_1$ die oben genannte Bedeutung haben und X für ein Chlor- oder Bromatom steht, mit einem Dioxolannylpiperidin der Formel III

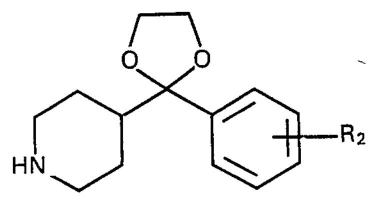

(III),

in welcher $R_2$ die oben genannte Bedeutung hat, umsetzt, und die erhaltene Verbindung der Formel IV

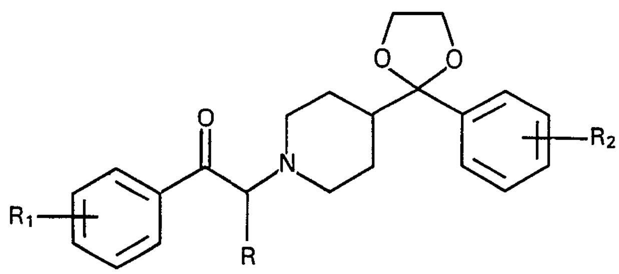

(IV)

einer Reduktion unterwirft, um eine Verbindung der Formel V

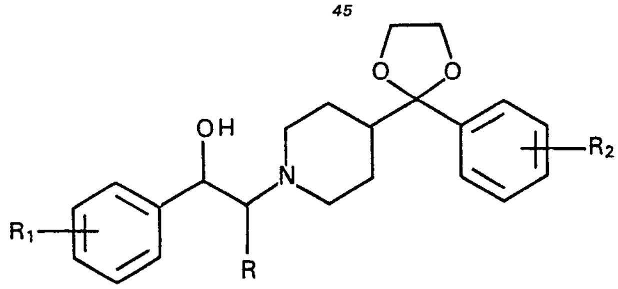

(V)

zu erhalten, die man schliesslich der Einwirkung einer Säure aussetzt.

2. Verfahren nach Anspruch 1, dadurch gekennzeichnet, dass man das halogenierte Keton (III) mit dem Dioxolannylpiperidin (IV) in Form des Salzes in einem Lösungmittel und in Gegenwart einer Base umsetzt.

3. Verfahren nach Anspruch 2, dadurch gekennzeichnet, dass man die Verbindung (IV) mit Hilfe eines Alkalimetallborhydrids reduziert.

4. Verfahren nach Anspruch 3, dadurch gekennzeichnet, dass man in Gegenwart von Essigsäure arbeitet.

5. Verfahren nach Anspruch 3, dadurch gekennzeichnet, dass man in Abwesenheit einer Säure arbeitet.

6. Verfahren zur Herstellung von Verbindungen, die der allgemeinen Formel I entsprechen

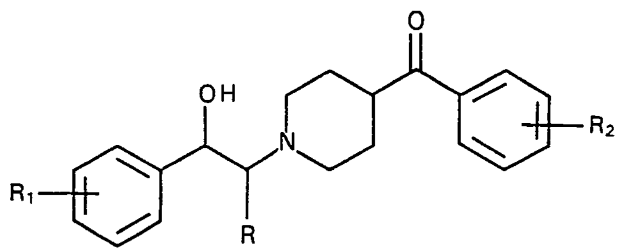

(I),

in welcher

R für ein Wasserstoffatom oder eine Methylgruppe,

$R_1$

– entweder für ein Wasserstoffatom oder einen einzigen Substituenten in der Stellung 2, 3 oder 4, wie ein Halogenatom, eine $C_{1-4}$-Alkyl-, $C_{1-4}$-Alkoxy-, Hydroxy-, Benzyloxy-, Trifluormethyl-, Cyano-, Nitro-, Amino-, Acetylamino-, Methylthio-, Methylsulfonyl- oder Aminosulfonylgruppe

– oder für insgesamt 3 Substituenten steht, die die Methoxygruppe in 4-Stellung und 2 Methylgruppen in den Stellungen 3 und 5 bedeuten,

und $R_2$

– entweder ein Wasserstoffatom oder einen einzigen Substituenten in der Stellung 3 oder 4, wie ein Fluor- oder Chloratom, eine Methylgruppe oder eine Methoxygruppe

– oder insgesamt 3 Substituenten darstellt, die 3 Methoxygruppen in den Stellungen 2, 4 und 6 bedeuten, welches Verfahren dadurch gekennzeichnet ist, dass man ein 4-Benzoylpiperidin der Formel VII

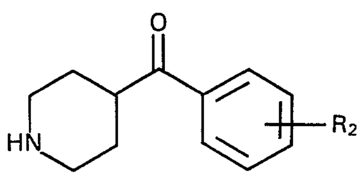

(VII),

in welcher $R_2$ die oben genannte Bedeutung hat, zu einem Epoxid der Formel VI

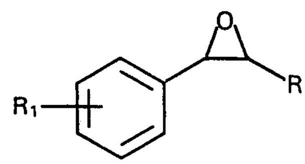

(VI)

in welcher R und $R_1$ die oben genannte Bedeutung haben, zusetzt.

**Claims for the Contracting States:**
BE, CH, DE, FR, GB, IT, LI, LU, NL, SE.

1. Compounds in the form of pure optical isomers or mixtures thereof, characterized in that they correspond to the general formula I

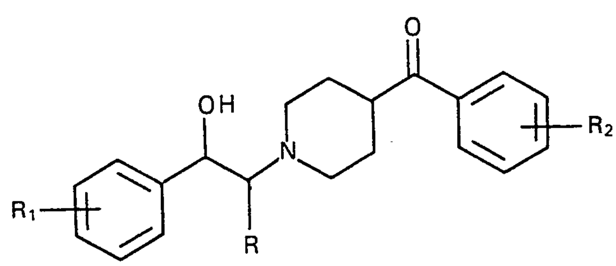

(I)

in which

R denotes a hydrogen atom or a methyl group,

$R_1$ denotes

– either a hydrogen atom or a single substituent at position 2, 3 or 4, such as a halogen atom or a $C_{1-4}$ alkyl, $C_{1-4}$ alkoxy, hydroxy, benzyloxy, trifluoromethyl, cyano, nitro, amino, acetylamino, methylthio, methylsulphonyl or aminosulphonyl group,

– or a combination of 3 substituents, namely, a methoxy group at position 4 and two methyl groups at positions 3 and 5, and

$R_2$ denotes

– either a hydrogen atom or a single substituent at position 3 or 4, such as a fluorine or chlorine atom, a methyl group or a methoxy group,

– or a combination of 3 substituents, namely, three methoxy groups at positions 2, 4 and 6,

as well as the addition salts thereof with pharmacologically acceptable acids.

2. Compounds according to Claim 1, characterized in that $R_1$ denotes a halogen atom or an alkyl group and $R_2$ denotes a fluorine atom.

3. Process for preparing the compounds according to Claim 1, characterized in that a halogenated ketone of formula II

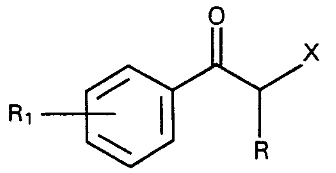

(II)

in which R and $R_1$ are as defined in Claim 1 and X denotes a chlorine or bromine atom, is reacted with a dioxolanylpiperidine of formula III

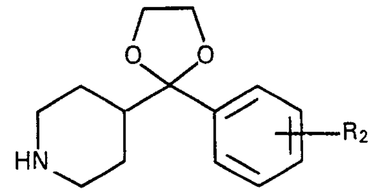

(III)

in which $R_2$ is as defined in Claim 1, and the compound obtained, of formula IV

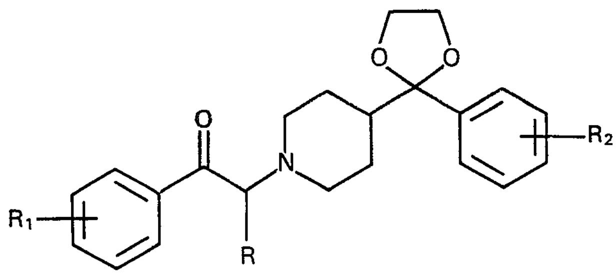

(IV)

is then subjected to reduction to obtain a compound of formula V

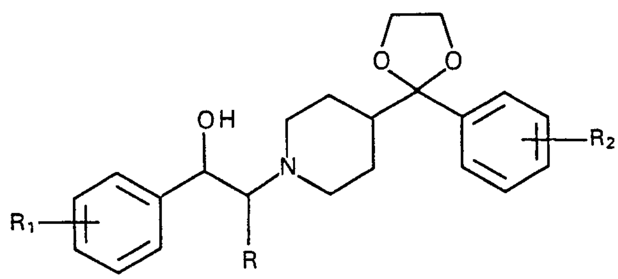

(V)

which is finally subjected to the action of an acid.

4. Process according to Claim 3, characterized in that the halogenated ketone (III) is reacted with the dioxolanylpiperidine (IV) in salt form, in a solvent and in the presence of a base.

5. Process according to Claim 3, characterized in that the compound (IV) is reduced by means of an alkali metal borohydride.

6. Process according to Claim 5, characterized in that the procedure is performed in the presence of acetic acid.

7. Process according to Claim 5, characterized in that the procedure is performed in the absence of acid.

8. Process for preparing the compound according to Claim 1, characterized in that a 4-benzoylpiperidine of formula VII

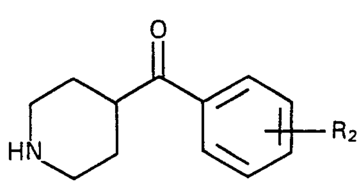

(VII)

in which $R_2$ is as defined in Claim 1, is added to an epoxide of formula VI

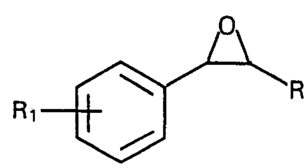

(VI)

in which R and $R_1$ are as defined in Claim 1.

9. Drug, characterized in that it consists of a compound according to one of Claims 1 and 2.

10. Pharmaceutical composition, characterized in that it contains a compound according to one of Claims 1 and 2, in combination with a suitable excipient.

**Claims for the Contracting State:** AT

1. Process for preparing compounds corresponding to the general formula I

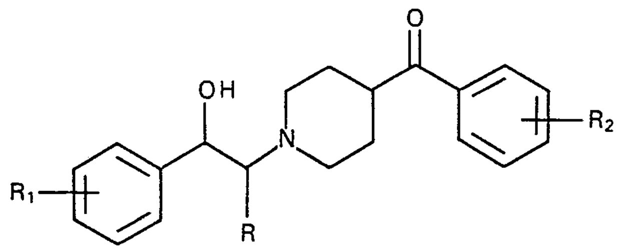

(I)

in which

R denotes a hydrogen atom or a methyl group,

$R_1$ denotes
– either a hydrogen atom or a single substituent at position 2, 3 or 4, such as a halogen atom or a $C_{1-4}$ alkyl, $C_{1-4}$ alkoxy, hydroxy, benzyloxy, trifluoromethyl, cyano, nitro, amino, acetylamino, methylthio, methylsulphonyl or aminosulphonyl group,
– or a combination of 3 substituents, namely, a methoxy group at position 4 and two methyl groups at positions 3 and 5, and
$R_2$ denotes
– either a hydrogen atom or a single substituent at position 3 or 4, such as a fluorine or chlorine atom, a methyl group or a methoxy group,
– or a combination of 3 substituents, namely, three methoxy groups at positions 2, 4 and 6,
which process is characterized in that a halogenated ketone of formula II

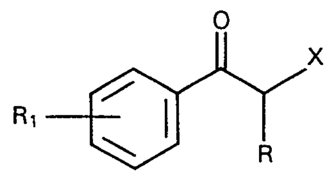

(II)

in which R and $R_1$ are as defined above and X denotes a chlorine or bromine atom, is reacted with a dioxolanylpiperidine of formula III

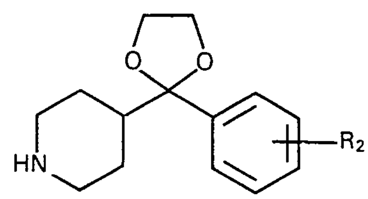

(III)

in which $R_2$ is as defined above, and the compound obtained, of formula IV

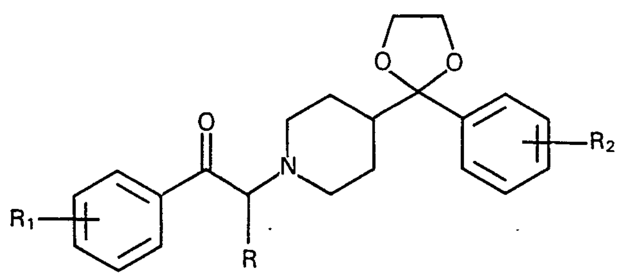

(IV)

is then subjected to reduction to obtain a compound of formula V

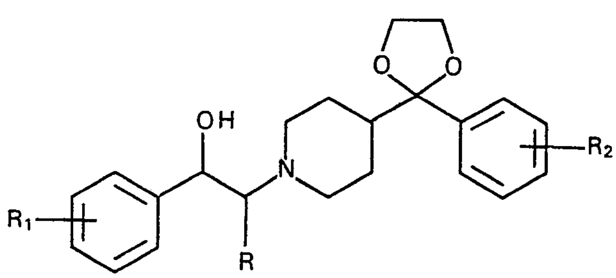

(V)

which is finally subjected to the action of an acid.

2. Process according to Claim 1, characterized in that the halogenated ketone (III) is reacted with the dioxolanylpiperidine (IV) in salt form, in a solvent and in the presence of a base.

3. Process according to Claim 2, characterized in that the compound (IV) is reduced by means of an alkali metal borohydride.

4. Process according to Claim 3, characterized in that the procedure is performed in the presence of acetic acid.

5. Process according to Claim 3, characterized in that the procedure is performed in the absence of acid.

6. Process for preparing compounds corresponding to the general formula I

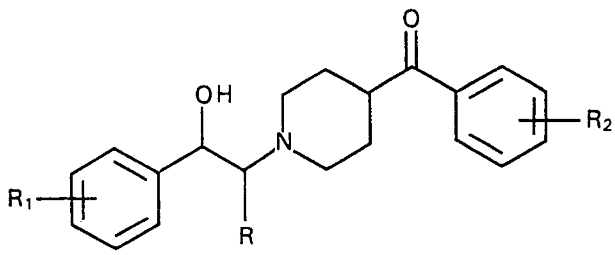

(I)

in which

R denotes a hydrogen atom or a methyl group,

$R_1$ denotes

– either a hydrogen atom or a single substituent at position 2, 3 or 4, such as a halogen atom or a $C_{1-4}$ alkyl, $C_{1-4}$ alkoxy, hydroxy, benzyloxy, trifluoromethyl, cyano, nitro, amino, acetylamino, methylthio, methylsulphonyl or aminosulphonyl group,

– or a combination of 3 substituents, namely, a methoxy group at position 4 and two methyl groups at positions 3 and 5, and

$R_2$ denotes

– either a hydrogen atom or a single substituent at position 3 or 4, such as a fluorine or chlorine atom, a methyl group or a methoxy group,

– or a combination of 3 substituents, namely, three methoxy groups at positions 2, 4 and 6,

which process is characterized in that a 4-benzoylpiperidine of formula VII

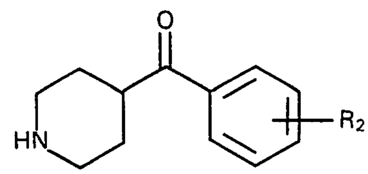

(VII)

in which $R_2$ is as defined above, is added to an epoxide of formula VI

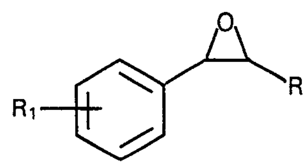

(VI)

in which R and $R_1$ are as defined above.